# EUROPEAN PATENT APPLICATION

(11) **EP 3 496 105 A1**
(43) Date of publication of application: **12.06.2019**
(21) Application number: 17206176.4
(22) Date of filing: 08.12.2017
(51) Int. Cl.: G16H 10/20, G16H 50/30, G16H 20/00

(54) **STATISTICAL ANALYSIS OF SUBJECT PROGRESS AND RESPONSIVE GENERATION OF INFLUENCING DIGITAL CONTENT**

(71) Applicant: Koninklijke Philips N.V., 5656 AE Eindhoven (NL)
(72) Inventor: CHEN, Huimin, 5656 AE Eindhoven (NL); YIN, Bin, 5656 AE Eindhoven (NL)
(74) Representative: de Haan, Poul Erik

(57) **Abstract**

Techniques disclosed herein relate to analysis of subject progress and responsive generation of influencing digital content. In various embodiments, a plurality of sets of data points pertaining to health of a subject may be received (402) via input component(s) of computing device(s). Weight(s) may be assigned (404) to the data point(s). A time series of progress scores associated with the subject may be determined (406) based on data points of the corresponding set of data points and weight(s). Various types of analysis, such as auto-regressive integrated moving average ("ARIMA") analysis, may be applied (408) to the time series of progress scores. Based on the analysis, future progress score(s) associated with the subject may be predicted (410). Influencing digital content may be generated/selected (412) based on the future progress score(s). The influencing digital content may be caused (414) to be presented to the subject via output component(s) of the computing device(s).

## Description

### FIELD OF THE INVENTION

Various embodiments described herein are directed generally to health care. More particularly, but not exclusively, various methods and apparatus disclosed herein relate to statistical analysis of subject progress and responsive generation of influencing digital content.

### BACKGROUND OF THE INVENTION

With the rise of aging populations, growing prevalence of chronic disease, and shortage of healthcare resources (e.g., doctors, clinicians, nurses, therapist, nutritionists, etc.), patent self-management at home and lifestyle management are taking on increasingly important roles. However, after discharge from a healthcare facility, many patients (or more generally, "subjects") often disconnect from their caregivers. Other than routine checkups at healthcare facilities, patients often have little to no contact with medical personnel, which means the medical personnel may lose track of the subjects' progress. For patients, several barriers hamper adequate self-management and correct lifestyle choices. One of the biggest barriers is insufficient health education. Medical personnel are extremely busy, making it impractical for them to spend enough time with each patient to provide comprehensive education and/or motivation. More generally, it is difficult for medical personnel to provide longitudinal and/or dynamic support that caters to each individual patient's needs and behavior. The predetermined care plan cannot be adjusted and updated iteratively according to different characteristics of sub-cohorts of patients.

### SUMMARY OF THE INVENTION

The present disclosure is directed to methods and apparatus for statistical analysis of subject progress and responsive generation of influencing digital content. For example, in various embodiments, a subject discharged from a healthcare facility may be provided with software, often referred to as an "app" in the context of mobile devices, that the subject can operate as time goes on to provide various data points pertaining to the subject's health. Some apps can additionally or alternatively acquire physiological data from physiological sensors such as blood pressure sensors, mobile electrocardiogram ("ECG"), step counters, etc., using communication technologies such as Bluetooth or Wi-Fi. These data points that pertain to the subject's health can include, for instance, information about the patient's medical records (e.g., medical history, diagnosis, demographics, etc.), vital signs (blood pressure, heart rate, ECG, etc.), habits (e.g., smoking, alcohol consumption), food intake (e.g., calories consumed, type of foods eaten, etc.), behavior (e.g., activity levels, types of activities, sedentary behavior, etc.), and so forth. Additionally or alternatively, in some embodiments, additional data points pertaining to the subject's health may be gathered automatically, e.g., from the subject's social media (e.g., status updates indicative of mood, health, activity engaged in, etc.), from physiological and/or motion sensors contained, for instance, in mobile computing devices carried by the subject, that may indicate activity levels and/or other physiological parameters, from digital images captured of food or other substance ingested by the subject, etc.

These data points pertaining to the subject's health may be collected over time and used to generate a time series of what will be referred to herein as "subject progress scores," or simply "progress scores" or "compliance scores." In some embodiments, each progress score of the time series of progress scores may be determined (e.g., calculated) using some weighted combination of individual data points obtained, for instance, during a particular time interval. For example, in some embodiments, a new progress score may be determined for a subject each day, each week, each month, etc. Additionally or alternatively, in some embodiments a new progress score may be determined when a sufficient number of data points, such as fifty, have been collected. The phrase "set of data points" is used herein to refer to a plurality of data points that are used to calculate a particular progress score. Thus, for instance, if progress scores are determined daily, then a particular set of data points that is used to determine a particular progress score of the time series of progress scores may include data points collected during a particular day.

Once a time series of progress scores is generated for a given subject, it may be statistically analyzed to predict one or more future progress scores associated with the given subject. In some embodiments, auto-regressive integrated moving average ("ARIMA"), ARIMAX (an extended ARIMA model with external regressor), autoregressive conditional heteroskedasticity model ("ARCH"), and/or other similar time-series analysis may be applied to the time series of progress scores to predict one or more future progress scores associated with the subject. This is one class of prediction models based on autocorrelation of data. However, other types of analysis, which may be implemented using machine learning/deep learning algorithms, may be employed, such as statistical classifiers (e.g., naive Bayes), support vector machines, random forest), neural networks, and so forth. Based on the one or more future progress scores, "influencing digital content" may be generated and caused to be presented to the subject via one or more output components of one or more computing devices operated by the subject. Meanwhile, in some embodiments, certain clinical action or intervention may be triggered if needed.

As a non-limiting example, in some embodiments, a clinician may examine the subject and, based on the examination, diagnose the subject with one or more health conditions. In various embodiments, influencing digital content that includes one or more questions or inquiries and corresponding educational material (related to wrong answers and wrong self-management behavior) may be generated and/or selected based on the diagnosis. These inquiries may, for instance, seek to ascertain one or more of the aforementioned data points that are pertinent to the subject's health. In particular, data points that are pertinent to the subject's diagnosis, including data points related to the patient's post-diagnosis habits and behavior, may be used to generate and/or select the questions. For example, a smoker may be asked how many cigarettes he or she smokes on average in a given day, or, as a question meant to educate, what is the harm caused by smoking. In some embodiments, the subject may be presented with the influencing digital content using an app installed, for instance, on the subject's smart phone or tablet computer. Depending on the subject's responses, the subject may be assigned an initial progress score (which may also be calculated based on other data points, such as data points gathered by the clinician during the examination, from sensors incorporated with the subject's mobile devices, etc.). In various embodiments, influencing digital content may additionally or alternatively include non-inquiry data, such as educational content that is generated and/or selected based on the subject's diagnosis, for presentation to the subject.

As time passes after the medical examination, the subject may be presented with additional influencing digital content on their app, to which they may provide responses that are used as subsequent data points. Additionally or alternatively, other subsequent data points pertinent to the subject's health, such as social media posts, sensor data from mobile device(s) operated by the subject, digital images of food, etc., may also be collected. In some embodiments, once enough subsequent data points are collected (and/or at the end of a given time interval, such as a day, a week, etc.), the subsequent data points may be used to determine a new progress score to add to a time series of progress scores associated with the patient. Then, statistical analysis, such as the aforementioned ARIMA analysis, may be applied to the time series of progress scores to predict future progress scores.

If it appears that the subject's overall progress will decline, then various remedial actions may be taken. In some embodiments, influencing digital content may be generated/selected and/or presented to the subject at an increased frequency, e.g., with the goal of influencing the subject to improve their behavior. In some embodiments, medical personnel may be informed of the predicted decline in progress, e.g., by way of output at one or more computing devices operated by the medical personnel, and the medical personnel may consult with the subject over the phone, in person, via email, etc. By contrast, if the subject is predicted to have future progress scores that represent an improvement, then influencing digital content may be generated/selected and/or presented at a lower frequency. Additionally or alternatively, in some embodiments, various rewards may be presented to the subject, such as a virtual trophy, pop-up message, avatar-based encouragement, etc.

Techniques described herein give rise to a number of technical advantages. Datadriven personalized educational content, quizzes, service and intervention are common in the telehealth industry. They enable better patient engagement based on individual data rather than population-level data. As another example, in some cases, throttling presentation of influencing digital content to subjects that appear to be improving may conserve computing resources such as processor cycles, memory, network bandwidth, etc., at one or more servers that generates and/or selects the influencing digital content and/or at individual client devices operated by the subject. Likewise, for subjects that appear to be falling back into bad habits, increasing a frequency at which influencing digital content is presented may reverse that trend preemptively by influencing the subjects to engage in healthier behavior before new bad habits form. This limits visits by the subject to medical personnel and/or potentially improves the subjects' health, lowering healthcare costs, not to mention transportation and other costs associated with subjects physically visiting medical facilities.

Moreover, prior attempts to present influencing digital content to subjects involved static and/or uniform presentation of influencing digital content to all subjects using healthcare software applications. Every subject was presented with graphical user interfaces that included the same or similar information, and these graphical user interfaces were presented without consideration of the subject's ongoing behavior. Thus, subjects that were actively trying to improve their behavior were nonetheless presented with influencing digital content, e.g., via graphical user interfaces, that they didn't need, making them less likely to engage with the software applications. Techniques described herein resolve these issues by ensuring that subjects are presented with influencing digital content at a frequency that is selected based on the subjects' behavior/habits. Consequently, subjects that improve their habits/behavior are less frequently disturbed or interrupted with unhelpful content, and subjects that are falling into bad habits and/or adopting unhealthy behaviors are increasingly influenced to improve.

Generally, in one aspect, a method may include: receiving, via one or more input components of one or more computing devices, a plurality of sets of data points pertaining to health of a subject; assigning one or more weights to one or more of the plurality of data points in each set of data points of the plurality of sets of data points; determining a time series of progress scores associated with the subject, wherein each progress score of the time series of progress scores is determined based on data points of the corresponding set of data points of the plurality of sets of data points and one or more of the weights; applying auto-regressive integrated moving average ("ARIMA") analysis to the time series of progress scores; predicting, based on the ARIMA analysis, one or more future progress scores associated with the subject; generating or selecting influencing digital content based on the one or more future progress scores; and causing the influencing digital content to be presented to the subject via one or more output components of one or more of the computing devices.

In various embodiments, each set of data points of the plurality of sets of data points may be associated with a time period that is distinct from time periods associated with other sets of the plurality of sets of data points. In various embodiments, the causing may include transmitting the influencing digital content to one or more of the computing devices over one or more networks. In various embodiments, at least one of the data points pertaining to the health of the subject may include input provided by the subject at one or more of the computing devices in response to an inquiry presented to the subject at one or more of the computing devices. In various embodiments, the inquiry relates to a habit of the subject and/or to nutritional intake of the subject.

In various embodiments, at least one of the data points pertaining to the health of the subject may include a digital photograph of food ingested by the subject, and the method may further include: performing image recognition of the digital photograph of food to assign one or more classifications to one or more food items ingested by the subject; and determining, as the at least one of the data points pertaining to the health of the subject, one or more food scores associated with the one or more assigned classifications.

In various embodiments, a frequency at which the influencing digital content is generated or selected, and caused to be presented to the subject, may be determined based on the ARIMA analysis. In various embodiments, at least one of the data points pertaining to the health of the subject may include content posted to social media by the subject. In various embodiments, at least one of the data points pertaining to the health of the subject may include activity data detected by one or more sensors of one or more of the computing devices that is carried by the subject while the subject engages in one or more physical activities. In various embodiments, at least one of the data points pertaining to the health of the subject may include one or more physiological parameters detected or measured by one or more physiological sensors of one or more of the computing devices.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

### BRIEF DESCRIPTION OF THE DRAWINGS

In the drawings, like reference characters generally refer to the same parts throughout the different views. Also, the drawings are not necessarily to scale, emphasis instead generally being placed upon illustrating various principles of the embodiments described herein.
Fig. 1 illustrates an example environment in which selected aspects of the present disclosure may be implemented, in accordance with various embodiments.
Fig. 2 depicts an example graphical user interface configured with various aspects of the present disclosure, in accordance with various embodiments.
Fig. 3 depicts another example graphical user interface configured with various aspects of the present disclosure, in accordance with various embodiments.
Fig. 4 depicts an example method for practicing selected aspects of the present disclosure.
Fig. 5 schematically depicts an example computer architecture, in accordance with various embodiments.

### DETAILED DESCRIPTION OF EMBODIMENTS

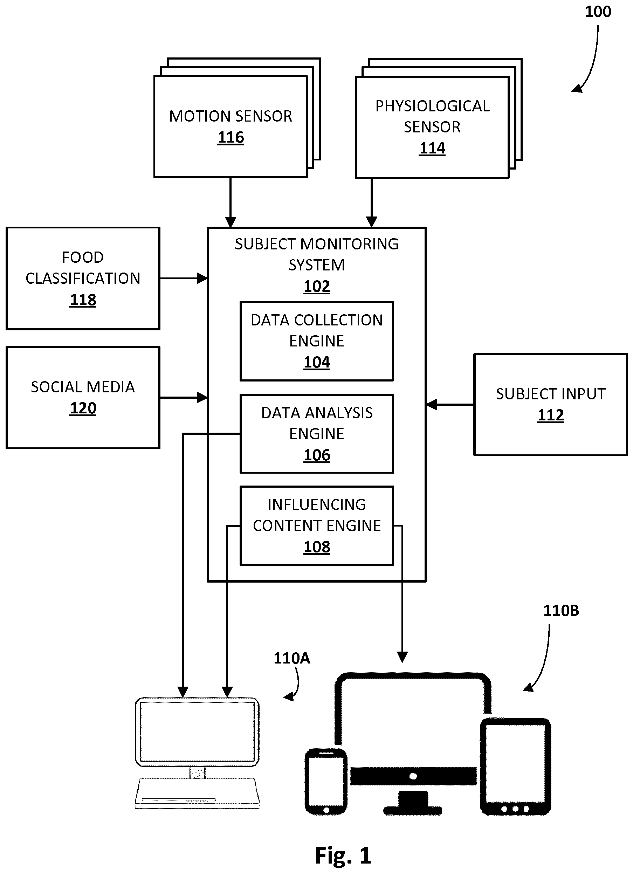

Fig. 1 illustrates an example environment 100 in which selected aspects of the present disclosure may be implemented, in accordance with various embodiments. A subject monitoring system 102 may be implemented on one or more computing systems (e.g., which may be connected via one or more computing networks), which in some cases may form what is sometimes referred to as the "cloud." Subject monitoring system 102 may include various modules and/or engines, any of which may be implemented using any combination of hardware or software. In Fig. 1, subject monitoring system 102 includes a data collection engine 104, a data analysis engine 106, and an influencing content engine 108. In other embodiments, one or more engines of subject monitoring system 102 may be omitted, combined with other engines/modules, or added.

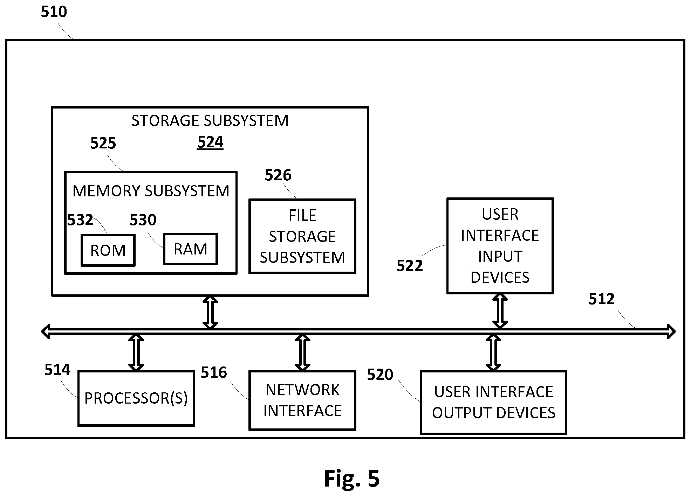

Subject monitoring system 102 may be operably coupled with one or more client devices 110 via one or more local area and/or wide are computing networks (not depicted), such as the Internet (which in some cases may connect subject monitoring system 102 to the so-call "cloud"). In Fig. 1, a first client device 110A takes the form of a computing device operated by one or more medical personnel, such as a clinician, nurse, therapist, caregiver, etc. One or more additional client devices 110B may be operated by one or more subjects, or patients, of the medical personnel. Client devices 110 operated by medical personnel and/or monitored subjects may come in various form factors, including but not limited to hand-held computing devices (e.g., smart phones, tablets), laptop computers, desktop computers, wearable computing devices (e.g., smart watches, smart glasses), standalone interactive speakers, smart televisions, and so forth. An example computing architecture that may be employed in any computing device mentioned herein to perform any aspect of the present disclosure is depicted in Fig. 5.

Generally speaking, subject monitoring system 102, e.g., by way of one or more of its constituent components (e.g., 104-108), may be configured to facilitate monitoring of subjects' progress post diagnosis/medical examination, and to facilitate performance of a variety of remedial actions designed to increase subjects' compliance with medical directives and to engage in lifestyle choices conducive to improved health. These remedial actions may include provision of influencing digital content to the subjects, e.g., as part of a healthcare software application that the subjects install on one or more computing devices they operate. For example, subjects may be presented with quizzes that ask questions about, for example, the subject's behavior/habits, the subject's knowledge about particular health issues, and so forth. These questions may seek not only to obtain data points pertinent to the subjects' health, but also to influence their future behavior. For example, asking subjects how many cigarettes they smoke each day forces them to consider their unhealthy habit, which may cause them to cut back or even quit. Subjects may also be presented with other influencing digital content, such as graphical user interfaces that display information meant to inform the subject about what they can do to improve their health outlook.

Data collection engine 104 may be configured to collect/receive/gather data points pertinent to subjects' health (which can include aspects such as psychology, behavior) from a variety of different data sources. In Fig. 1, these data sources include subject input 112 obtained, for instance, in response to presentation of the aforementioned influencing digital content. For example, the subject may be asked questions about their lifestyle, habits, activity levels, etc. Their answers may be used as data points that subject monitoring system 102 may use to practice techniques described herein.

Data collection engine 104 may additionally or alternatively collect one or more data points pertinent to subjects' health from one or more physiological sensors 114. Physiological sensor(s) 114 may include, for example, glucose meters, blood pressure monitors, photoplethysmogram ("PPG") sensors, and sensors that measure/detect other physiological parameters. Some physiological sensors 114 may be deployed on mobile devices commonly used by subjects, such as smart watches, smart phones, smart glasses, etc. For example, many smart phones and smart watches include heart rate sensors. Other physiological sensors may be deployed as standalone medical devices. For example, diabetic subjects may carry electronic glucose readers that may provide, to data collection engine 104 as data points pertinent to subjects' health, glucose readings. In some embodiments, a standalone medical device may be "paired" with a more conventional mobile device such as a smart phone such that sensor readings are transmitted from the standalone medical device to the smart phone (e.g., using wireless technologies such as Bluetooth or Wi-Fi), which in turn may transmit data indicative of the sensor readings to subject monitoring system 102. In other embodiments, standalone electronic medical devices may provide data points directly to subject monitoring system 102.

Data collection engine 104 may additionally or alternatively collect one or more data points pertinent to subjects' health (including psychology and behavior) from one or more motion sensors 116. Motion sensor(s) 116 may be configured to measure and/or detect various types of motion undergone by subjects. For example, position coordinate sensors such as Global Positioning System ("GPS") sensors may be configured to provide information about distances travelled, e.g., by subjects who exercise by walking, running, riding a bicycle, etc. From a psychology perspective, decreased physical activity might be an indication of depression. Additionally or alternatively, accelerometers or other similar sensors (e.g., gyroscopes) may provide data indicative of, for instance, aerobic exercise, steps taken, etc. As was the case with physiological sensor(s) 114, motion sensor(s) 116 may be deployed on conventional mobile devices operated by subjects, or as standalone devices that, for example, wirelessly communicate with mobile devices operated by subjects.

In some embodiments, data collection engine 104 may obtain data points pertinent to subjects' health, psychology and/or behavior in the form of food classifications. For example, subjects may, e.g., via the health application described previously, fill out a "food diary" in which the subjects provide information about what food they ingested. In some embodiments, subjects may be able to capture digital images of food they are about to eat, e.g., using mobile phone cameras. In some such embodiments, various object recognition techniques, including but not limited to one or more trained convolutional neural networks, may be employed, e.g., at a mobile device carried by the subject, at subject monitoring system 102, and/or at one or more other cloud-based components, to assign classifications to foods that the subject is about to eat. Data indicative of these food classifications may be provided to data collection engine 104, e.g., as additional data points pertinent to subjects' health. Convolutional neural networks and other techniques for object recognition using digital photographs are known in the art, but application of those techniques in the context of the present disclosure is not known.

In some embodiments, data collection engine 104 may acquire data points from social media 120 engaged in by subjects. For example, in some embodiments, data collection engine 104 may utilize various known natural language processing techniques (e.g., topic classifiers) to determine whether a subject's social media post (e.g., status update, uploaded digital image) relates to health, exercise or mood. If the post is unrelated to subject health, it may be discarded. But if the post relates to subject health-e.g., "I had a great time running the Hypothetical 5K today!"-then data collection engine 104 may consider at least some content of the post (e.g., action=run, object=five kilometers) a data point pertinent to the subject's health. The data sources depicted in Fig. 1 are not meant to be limiting. Any number of other data sources may be used to obtain data points pertinent to subjects' health, psychology and/or behavior.

Data analysis engine 106 may be configured to perform various types of calculations based on data points collected by data collection engine 104. For example, data analysis engine 106 (or data collection engine 104) may be configured to organize data points into temporally distinct sets of data points, e.g., wherein each set corresponds at least generally to some time interval (e.g., a day, a week, an hour, etc.). In some embodiments, data points may be strictly organized into sets based on a time interval in which they were acquired. For example, all data points obtained on Tuesday may be organized into a set of data points associated with Tuesday, whereas all data points obtained on Wednesday may be organized into a set of data points associated with Wednesday. Additionally or alternatively, in some embodiments, data points are organized into distinct sets of data points such that each distinct set includes at least a minimum number of data points. For example, in some embodiments, a first set may include fifty consecutively-received data points, a second set that immediately follows the first set may include the next fifty consecutively-received data points, and so on. In some embodiments this may mean that one set of data points may include data points acquired over a longer time interval than another set of data points. However, in general, the sequence of sets of data points may essentially form a time series of sets of data points, with one set following another in a generally temporal fashion. Additionally or alternatively, in some embodiments, some data, such as blood pressure, heart rate, detected physical activity, etc. may be collected on a relatively frequent basis, such as daily. Other data, such as diagnoses, medication prescription(s), quality of life questionnaire answers, etc., may be collected on a less frequent basis, such as weekly, monthly, and/or as needed.

Data analysis engine 106 may also be configured to determine time series of progress scores associated with subjects. Each progress score of each time series of progress scores may be determined, e.g., by data analysis engine 106, based on data points of a corresponding set of data points of the plurality of sets of data points. Thus, data points in a "Tuesday" set of data points associated with a subject may be used to calculate a "Tuesday" progress score associated with the subject. Data points in a "Wednesday" set of data points associated with the subject may be used to calculate a "Wednesday" progress score associated with the subject. Data points in a "Thursday" set of data points associated with the subject may be used to calculate a "Thursday" progress score associated with the subject. And so on. Progress scores may take various forms, such as integers, real numbers, numbers along a predetermined scale, and so forth.

In some embodiments, when calculating progress scores, individual data points pertaining to subjects' health, psychology, and/or behavior may be weighted in various ways, e.g., to reflect the relative importance of the data points to subjects' health. For example, the number of cigarettes smoked in a day may be weighted more heavily than, for instance, the number of steps taken in the same day. This may be particularly true where a particular data point is especially relevant to a subject's diagnosis. For example, the number of cigarettes smoked may be weighted more heavily for a subject diagnosed with lung cancer or chronic obstructive pulmonary disease ("COPD") than for a patient with no such diagnosis (e.g., a subject that only smokes occasionally). As another example, a relatively high glucose reading may be weighted more heavily for a diabetic subject than, say, another subject with no history of diabetes. More generally, various data points may be weighted based on their relative importance to subjects' health. Weights assigned to data points associated with a subject may be selected manually by medical personal or automatically, e.g., by data analysis engine 106.

Once a time series of progress scores is established for a subject, data analysis engine 106 may be configured to apply various types of statistical analysis to the time series of progress scores to predict one or more future progress scores associated with the subject. For example, in some embodiments, data analysis engine 106 may apply auto-regressive integrated moving average ("ARIMA") analysis to the time series of progress scores to predict one or more future progress scores. ARIMA analysis fits a model to time series data to, among other things, predict (or "forecast") future points in the time series. For example, given a time series of progress scores, the "integration" step of ARIMA analysis may be performed to make the data "stationary" (e.g., joint probability distribution does not change when shifted in time). For example, the data values (progress scores in the present context) may be replaced with the difference between their values and the previous values (this differencing process may be iterated one or more times as needed). The "autoregressive" aspect of ARIMA analysis relates to how the evolving variable of interest (in this present context, the progress score) is regressed on its own lagged, or prior, values. The "moving average" aspect of ARIMA analysis indicates that the regression error is a linear combination of error terms. In some embodiments, ARIMAX, the extended ARIMA model, may be used to include the impact of external factor(s). For example, temperature may have influence on blood pressure, physical activity, mood and etc.

Additionally or alternatively, other types of statistical analysis, which may or may not be implemented using machine learning algorithms, may be employed, such as statistical classifiers (e.g., naive Bayes), support vector machines, random forest, neural networks, and so forth. In some embodiments, the data points of a given set of data points associated with a given time interval may be organized into a feature vector that is then applied as input across one or more of the aforementioned machine learning classifiers to generate output. In some embodiments the output may be indicative of a progress score. The reliability of the predicted future progress scores may depend on various factors, such as how far in the future the scores are predicted, the amount of data points that were used to calculate the current progress scores, etc.

Influencing content engine 108 may be configured to generate or select (e.g., from a library) influencing digital content based on the one or more future progress scores predicted by data analysis engine 106. In various embodiments, influencing content engine 108 may further be configured to cause influencing digital content to be presented to subjects via one or more output components of client devices 110B. In some embodiments, influencing content engine 108 may generate and/or select, for presentation to subjects, influencing digital content at a frequency that is selected based on predicted future progress scores for the subjects. For example, suppose a given subject is predicted to have a decline in progress scores. Influencing content engine 108 may cause influencing digital content to be presented to the subject at an increased frequency, in the hopes of influencing the subject to engage in healthier behavior.

In some embodiments, medical personnel may be able to access, e.g., by way of operating client device(s) 110A, the future progress scores predicted by data analysis engine 106. Additionally or alternatively, in some embodiments, influencing content engine 108 may provide notification to medical personal, e.g., via client device(s) 110A (e.g., by way of text message, email, graphical user interface of a software application, etc.), of predicted future progress scores. Either way, the medical personnel may be able to take remedial action, such as consulting with the patient over the telephone or in person, and/or by causing influencing content engine 108 to present influencing digital content selected by the medical personnel to the subject. In some embodiments, medical personnel may be able to operate a graphical user interface to drill down into the future progress scores, e.g., by analyzing individual data points and/or their assigned weights, to identify a specific underlying cause to a predicted change in future progress scores. For example, a clinician may be able to examine a particular data point associated with each progress score of the time series of progress scores-say, number of cigarettes smoked in a day-to determine whether that data point is a primary cause for concern.

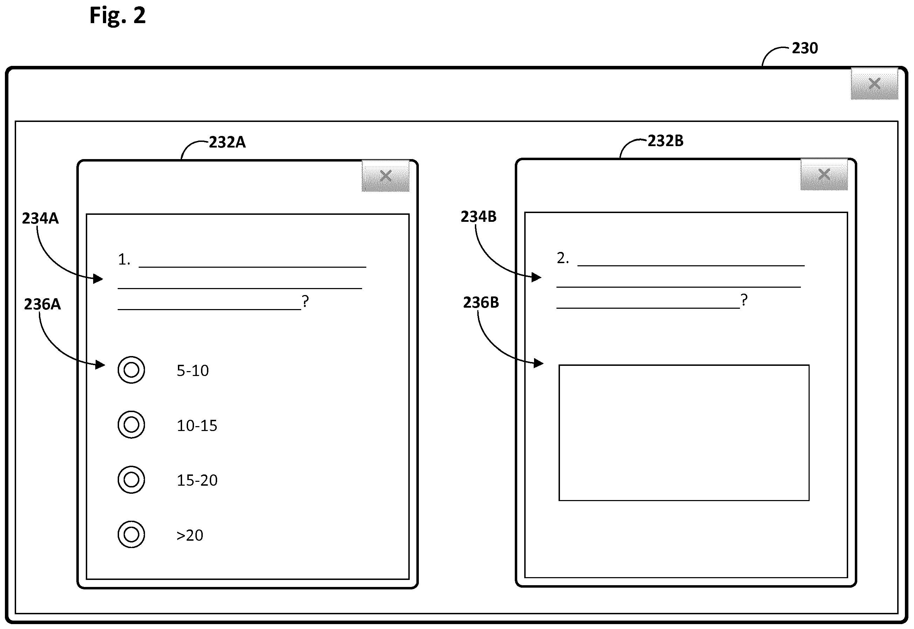

Fig. 2 schematically depicts an example graphical user interface ("GUI") 230 that may be presented to a subject, e.g., on one or more client devices 110A, in accordance with various embodiments. GUI 230 may be associated with the healthcare software application described above that may be used by subjects to provide data and receive influencing digital content. Additionally or alternatively, GUI 230 may be associated with a web browser that renders visible content based on underlying markup language (e.g., HTML, XML). GUI 230 of Fig. 2 is of a type that typically would be presented on a display screen associated with a laptop or desktop computer. However, this is not meant to be limiting, and similar graphical elements (e.g., influencing digital content) may be presented on a GUI tailored to mobile devices, including smart phones, smart watches, smart glasses, augmented reality headsets, etc.

GUI 230 in this example is be rendered to present two instances of influencing digital content, 232A and 232B, to the subject/user. More or less instances of influencing digital content may be presented simultaneously, and/or sequentially, over time. First influencing digital content 232A takes the form of a pop-up window or mini-GUI that presents a first question 234A that solicits one or more data points pertaining to the subject's health. In this example, the subject's response is constrained to an enumerated list 236A of answers (from which the subject may select by clicking a radio button).

In some embodiments, first question 234A may seek information such as how many cigarettes the subject smoked in the current day, the last twenty four hours, etc. In this example, the subject may select from enumerated list 236A an answer that accurately indicates a range of cigarettes smoked. Other similar questions might ask how far the subject walked/ran or rode a bike, how long the subject engaged in cardiovascular activity, how many alcoholic beverages the subject consumed, what the subject's blood glucose reading is, what the subject's blood pressure is, etc.

Second influencing digital content 232B includes a second question 234B and then an answer space 236B in which the subject can provide a free-form response. In some embodiments, various natural language processing techniques may be employed on such free form responses to detect data points pertinent to the subject's health. Examples of questions that might be used in such an open-ended context include but are not limited to "How are you feeling today," "What is currently bothering you," "What do you know about managing blood sugar," etc.

Influencing digital contents 232A and 232B are presented as pop-up windows in Fig. 2. However, this is not meant to be limiting. In various embodiments, influencing digital content may be presented in other forms. For example, in some embodiments, influencing digital content may simply be presented as part of a "home screen" rendered in GUI 230, whether GUI 230 is associated with a custom software application or accessed as a webpage. In Fig. 2, the subject is presented with two instances of influencing digital content, e.g., upon the subject launching GUI 230. This may be because techniques described herein predicted that the subject is predicted to undergo a downward trend in progress scores. Other subjects that are predicted to undergo steady or even improving trends may be presented with less influencing digital content.

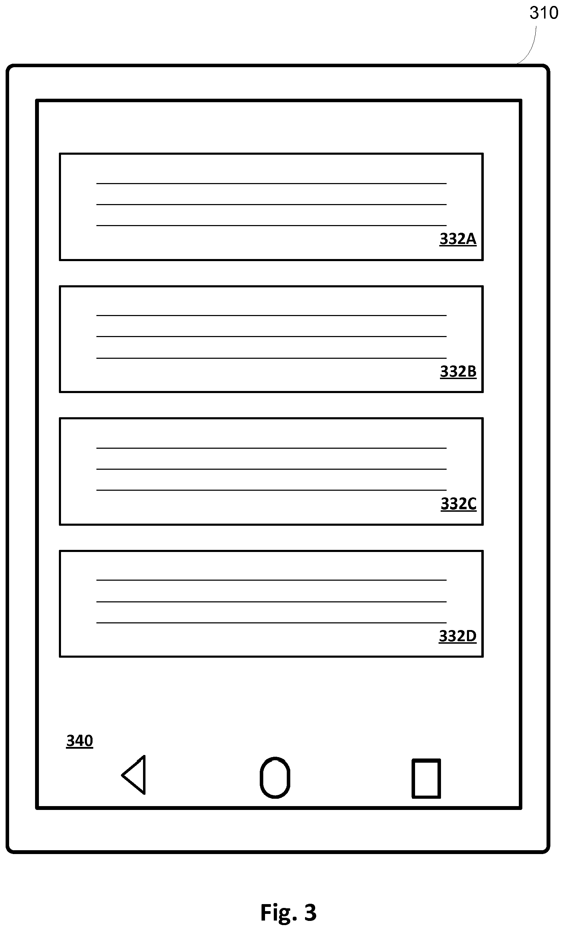

Fig. 3 depicts another example of how influencing digital content may be presented to a subject. In the example of Fig. 3, a client device 310 in the form of a smart phone or tablet with a touchscreen 340 is depicted that may be operated, for instance, by the subject. Suppose for this example that the subject is predicted to undergo a downward trend in progress scores. As a consequence, this subject is presented with four instances of influencing digital content 332A-D, which in this example take the form of notifications that appear on the subject's home and/or lock screen. These notification may appear simultaneously and/or may accumulate on the subject's screen over time (e.g., until the subject dismisses or otherwise engages with the notifications). In various embodiments, the notifications may simply be informative (e.g., provide a health tip) or may be interactive, e.g., such that the subject can select a notification to, for instance, navigate a web browser or another software application to a particular webpage or GUI, or to answer a health question. In some embodiments, if the subject wishes to dismiss the notifications, they may "swipe right" or perform some other similar action. Another subject predicted to undergo a less negative progress score trend may be presented with less notifications.

In other embodiments, influencing digital content may be presented in other manners. For example, with a standalone interactive speaker or vehicle computing system, the subject may be presented influencing digital content in audible form, e.g., by way of a personal voice assistant or "chatbot." For example, when a subject first vocally engages with a standalone interactive speaker in the morning, the personal voice assistant may, e.g., after responding to any of the subject's natural language requests, proactively provide, e.g., as natural language output, various influencing digital content, such as questions about the subject's habits (to which the subject may provide natural language responses), health tips that are selected based on the subject's particular diagnosis, etc. In some embodiments in which the subject operates a coordinated "ecosystem" of client devices, influencing digital content may be presented on whichever client device of the ecosystem the subject engages with first after the digital content is pushed to the subject. In some embodiments, the output modality selected for presentation of influencing digital content may depend on a variety of factors, such as the capabilities of the client device operated by the subject (e.g., an interactive speaker is only capable of providing audio output), the subject's preferences, etc. In some embodiments, influencing digital content may be pushed to subjects using techniques that make the influencing digital content available on multiple types of client devices, such as by sending text messages, emails, voicemails, messages on social media feeds, etc. In some embodiments, clinical service or intervention may be triggered if needed.

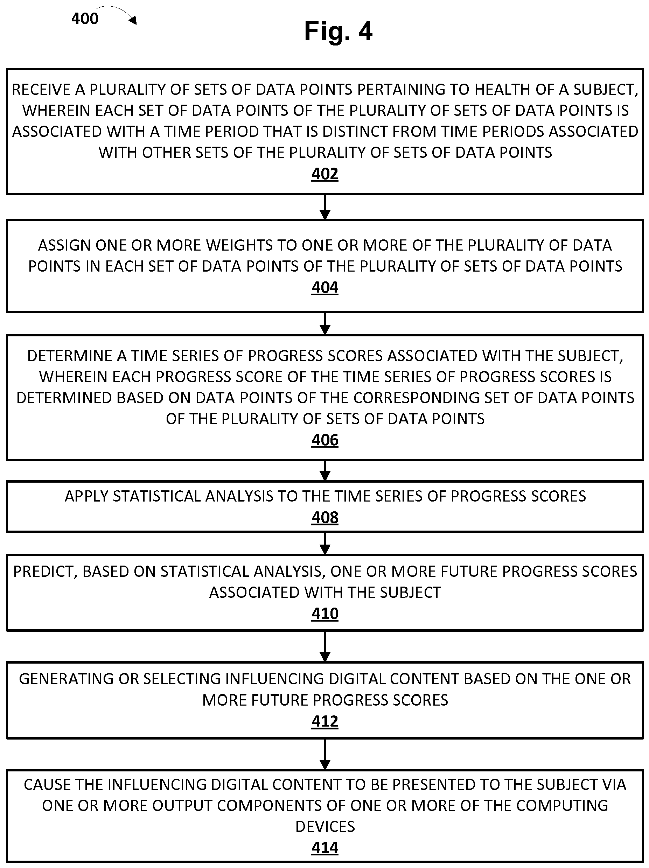

Fig. 4 depicts an example method 400 for practicing selected aspects of the present disclosure, in accordance with various embodiments. For convenience, the operations of the flow chart are described with reference to a system that performs the operations. This system may include various components of various computer systems, including those operating subject monitoring system 102 and/or its constituent components. Moreover, while operations of method 400 are shown in a particular order, this is not meant to be limiting. One or more operations may be reordered, omitted or added.

At block 402, the system may receive, e.g., from one or more of data sources 112-120 in Fig. 1, a plurality of sets of data points pertaining to health of a subject. In some embodiments, each set of data points of the plurality of sets of data points may be associated with a time period that is distinct from time periods associated with other sets of the plurality of sets of data points. For example, each set may correspond to data points gathered during a particular day, week, month, etc. Additionally or alternatively, in some embodiments, data points may be gathered until some minimum (e.g., 50) number of data points is gathered, and/or until some enumerated list of specific required data points are gathered. For example, for a subject diagnosed with COPD, required data points may include number of cigarettes smoked, distance walked, breathing exercises performed, medications taken, etc. For subjects using the app below certain threshold of usage time, pre-determined influencing digital content may be provided.

At block 404, the system may assign one or more weights to one or more of the plurality of data points in each set of data points of the plurality of sets of data points. As noted above, these weights may be selected automatically, e.g., by subject monitoring system 102, or manually, e.g., by medical personnel. Weights assigned to data points may be selected for a variety of reasons, including the relative importance of a given data point to a particular patient's health, new knowledge learned about a given condition (e.g., therapeutic breakthroughs, new research, etc.), and so forth.

At block 406, the system may determine a time series of progress scores associated with the patient. Each progress score of the time series of progress scores may be determined, for instance, based on data points of the corresponding set of data points of the plurality of sets of data points and one or more of the weights assigned at block 404. Thus, if a set of data points includes data points gathered during a particular day while another set of data points from the same subject gathered on a weekly/monthly/other basis, then the progress score determined based on these sets may be associated with the same particular day. For example, if questionnaire of quality of life, S_{Q} is collected every two months, then S_{Q} may be the same for consecutive sixty-day periods.

At block 408, the system may apply statistical analysis, such as ("ARIMA") analysis, to the time series of progress scores. At block 410, the system may predict, based on the statistical analysis, one or more future progress scores associated with the subject. These future progress scores may reflect a prediction that the subject is likely to undergo an increase in progress scores (i.e., the patient appears to be improving his or her behavior), a prediction that the subject is maintaining steady progress scores (i.e., the patient is at least not falling back into bad habits), and/or a prediction that the subject is likely to undergo a decline in progress scores (i.e., the patient is falling into bad habits).

At block 412, the system may generate and/or select influencing digital content based on the one or more future progress scores. For example, in some embodiments, a library of preexisting influencing digital content may be available from which the system can select suitable influencing digital content that is determined to be pertinent to a particular subject based on, for instance, the subject's diagnosis and/or a drill down to reveal individual factors that lead to a downward trend in the subject's progress scores. Additionally or alternatively, in some embodiments the system may generate influencing digital content on the fly, e.g., based on output modalities available on client devices most often used by the subject. For example, if it is determined that a subject most often interacts with a given client device (e.g., a vehicle computing system), influencing digital content may be selected and/or generated that is most suitable for presentation over an output modality available to that device. In other words, in some embodiments the system may select/generate influencing digital content in a manner that is most likely to be consumed by the subject, in order to have the greatest influence on the subject's behavior.

At block 414, the system may cause the influencing digital content to be presented to the subject via one or more output components of one or more of the computing devices. Again, which output component of which client device may be selected based on a number of factors, such as which client device is used most often by the subject, which client device is usually used first by the subject in a given day, which client device has an output modality most suitable for presentation of the influencing digital content, etc. In many embodiments, the client device may be remote from subject monitoring system 102. Accordingly, in some such embodiments, the operations of block 414 may include transmitting information indicative of the influencing digital content over one or more computing networks to the subject's client device. As noted above, the operations of blocks 412-414 may in some embodiments be performed at a frequency that is selected based on the one or more future progress scores. If a subject is predicted to undergo a downward trend in progress scores, they may receive influencing digital content more frequently. If a subject is predicted to remain steady or improve, provision of influence content to that subject may be throttled.

Fig. 5 is a block diagram of an example computing device 510 that may optionally be utilized to perform one or more aspects of techniques described herein. Computing device 510 typically includes at least one processor 514 which communicates with a number of peripheral devices via bus subsystem 512. These peripheral devices may include a storage subsystem 524, including, for example, a memory subsystem 525 and a file storage subsystem 526, user interface output devices 520, user interface input devices 522, and a network interface subsystem 516. The input and output devices allow user interaction with computing device 510. Network interface subsystem 516 provides an interface to outside networks and is coupled to corresponding interface devices in other computing devices.

User interface input devices 522 may include a keyboard, pointing devices such as a mouse, trackball, touchpad, or graphics tablet, a scanner, a touchscreen incorporated into the display, audio input devices such as voice recognition systems, microphones, and/or other types of input devices. In general, use of the term "input device" is intended to include all possible types of devices and ways to input information into computing device 510 or onto a communication network.

User interface output devices 520 may include a display subsystem, a printer, a fax machine, or non-visual displays such as audio output devices. The display subsystem may include a cathode ray tube (CRT), a flat-panel device such as a liquid crystal display (LCD), a projection device, or some other mechanism for creating a visible image. The display subsystem may also provide non-visual display such as via audio output devices. In general, use of the term "output device" is intended to include all possible types of devices and ways to output information from computing device 510 to the user or to another machine or computing device.

Storage subsystem 524 stores programming and data constructs that provide the functionality of some or all of the modules described herein. For example, the storage subsystem 524 may include the logic to perform selected aspects of the method of Fig. 4, as well as to implement various components depicted in Fig. 1.

These software modules are generally executed by processor 514 alone or in combination with other processors. Memory 525 used in the storage subsystem 524 can include a number of memories including a main random access memory (RAM) 530 for storage of instructions and data during program execution and a read only memory (ROM) 532 in which fixed instructions are stored. A file storage subsystem 526 can provide persistent storage for program and data files, and may include a hard disk drive, a floppy disk drive along with associated removable media, a CD-ROM drive, an optical drive, or removable media cartridges. The modules implementing the functionality of certain implementations may be stored by file storage subsystem 526 in the storage subsystem 524, or in other machines accessible by the processor(s) 514.

Bus subsystem 512 provides a mechanism for letting the various components and subsystems of computing device 510 communicate with each other as intended. Although bus subsystem 512 is shown schematically as a single bus, alternative implementations of the bus subsystem may use multiple busses.

Computing device 510 can be of varying types including a workstation, server, computing cluster, blade server, server farm, or any other data processing system or computing device. Due to the ever-changing nature of computers and networks, the description of computing device 510 depicted in Fig. 5 is intended only as a specific example for purposes of illustrating some implementations. Many other configurations of computing device 510 are possible having more or fewer components than the computing device depicted in Fig. 5.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03. It should be understood that certain expressions and reference signs used in the claims pursuant to Rule 6.2(b) of the Patent Cooperation Treaty ("PCT") do not limit the scope.

## Claims

1. A method implemented by one or more processors, comprising:
receiving (402), via one or more input components of one or more computing devices, a plurality of sets of data points pertaining to health of a subject;
assigning (404) one or more weights to one or more of the plurality of data points in each set of data points of the plurality of sets of data points;
determining (406) a time series of progress scores associated with the subject, wherein each progress score of the time series of progress scores is determined based on data points of the corresponding set of data points of the plurality of sets of data points and one or more of the weights;
applying (408) auto-regressive integrated moving average ("ARIMA") analysis to the time series of progress scores;
predicting (410), based on the ARIMA analysis, one or more future progress scores associated with the subject;
generating or selecting (412) influencing digital content based on the one or more future progress scores; and
causing (414) the influencing digital content to be presented to the subject via one or more output components of one or more of the computing devices.

2. The method of claim 1, wherein each set of data points of the plurality of sets of data points is associated with a time period that is distinct from time periods associated with other sets of the plurality of sets of data points.

3. The method of claim 1, wherein the causing comprises transmitting the influencing digital content to one or more of the computing devices over one or more networks.

4. The method of claim 1, wherein at least one of the data points pertaining to the health of the subject includes input provided by the subject at one or more of the computing devices in response to an inquiry presented to the subject at one or more of the computing devices.

5. The method of claim 4, wherein the inquiry relates to a habit of the subject.

6. The method of claim 4, wherein the inquiry relates to nutritional intake of the subject.

7. The method of claim 1, wherein at least one of the data points pertaining to the health of the subject comprises a digital photograph of food ingested by the subject, and the method further comprises:
performing image recognition of the digital photograph of food to assign one or more classifications to one or more food items ingested by the subject; and
determining, as the at least one of the data points pertaining to the health of the subject, one or more food scores associated with the one or more assigned classifications.

8. The method of claim 1, wherein a frequency at which the influencing digital content is generated or selected, and caused to be presented to the subject, is determined based on the ARIMA analysis.

9. The method of claim 1, wherein at least one of the data points pertaining to the health of the subject comprises content posted to social media by the subject.

10. The method of claim 1, wherein at least one of the data points pertaining to the health of the subject comprises activity data detected by one or more sensors of one or more of the computing devices that is carried by the subject while the subject engages in one or more physical activities.

11. The method of claim 1, wherein at least one of the data points pertaining to the health of the subject comprises one or more physiological parameters detected or measured by one or more physiological sensors of one or more of the computing devices.

12. A system comprising one or more processors and memory operably coupled with the one or more processors, wherein the memory stores instructions that, in response to execution of the instructions by one or more processors, cause the one or more processors to perform the following operations:
receiving (402), via one or more input components of one or more computing devices, a plurality of sets of data points pertaining to health of a subject;
assigning (404) one or more weights to one or more of the plurality of data points in each set of data points of the plurality of sets of data points;
determining (406) a time series of progress scores associated with the subject, wherein each progress score of the time series of progress scores is determined based on data points of the corresponding set of data points of the plurality of sets of data points and one or more of the weights;
applying (408) auto-regressive integrated moving average ("ARIMA") analysis to the time series of progress scores;
predicting (410), based on the ARIMA analysis, one or more future progress scores associated with the subject;
generating or selecting (412) influencing digital content based on the one or more future progress scores; and
causing (414) the influencing digital content to be presented to the subject via one or more output components of one or more of the computing devices.

13. At least one non-transitory computer-readable medium comprising instructions that, in response to execution of the instructions by one or more processors, cause the one or more processors to perform the following operations:
receiving (402), via one or more input components of one or more computing devices, a plurality of sets of data points pertaining to health of a subject;
assigning (404) one or more weights to one or more of the plurality of data points in each set of data points of the plurality of sets of data points;
determining (406) a time series of progress scores associated with the subject, wherein each progress score of the time series of progress scores is determined based on data points of the corresponding set of data points of the plurality of sets of data points and one or more of the weights;
applying (408) auto-regressive integrated moving average ("ARIMA") analysis to the time series of progress scores;
predicting (410), based on the ARIMA analysis, one or more future progress scores associated with the subject;
generating or selecting (412) influencing digital content based on the one or more future progress scores; and
causing (414) the influencing digital content to be presented to the subject via one or more output components of one or more of the computing devices.
